# EUROPEAN PATENT APPLICATION

(11) **EP 4 733 768 A2**
(43) Date of publication of application: **29.04.2026**
(21) Application number: 25221989.4
(22) Date of filing: 08.12.2022
(51) Int. Cl.: G01N 33/68

(54) **A METHOD OF PROVIDING PEPTIDES**

(30) Priority: 09.12.2021 GB 202117821
(62) Divisional of application: 22838911.0
(71) Applicant: The University Court Of The University of Edinburgh, Edinburgh EH8 9YL (GB)
(72) Inventor: GILBERT, Nick, Edinburgh, EH4 2XU (GB); KLEINJAN, Dirk, Edinburgh, EH9 3BF (GB); MARSH, Joseph, Edinburgh, EH4 2XU (GB); KEARNS, Patrick, Edinburgh, EH4 2XU (GB)
(74) Representative: Marks & Clerk LLP

(57) **Abstract**

A method of identifying stable peptides from a protein, said method comprising: obtaining a peptide from a protein; comparing a solvent-accessible surface area (SASA) of the peptide with a SASA of a corresponding peptide region within the protein; using a result of the comparison to determine whether or not the peptide is structurally stable relative to the corresponding peptide region within the protein. Also disclosed is use of the method to identify stable immunogenic epitopes of SARS-CoV-2, and methods of detecting an antibody response.

## Description

### FIELD

The present disclosure relates to methods of thermodynamic prediction, synthesis and prioritisation of immunogenic peptides, to immunogenic peptides prepared by such methods and uses of the same

### BACKGROUND

Proteins may exhibit complex three-dimensional (3D) structures and surface exposed amino acids that, when injected into an animal such as a human, a mouse, or a rabbit, may trigger an immune response resulting in the generation of antibodies to specific protein epitopes.

It is desirable to identify parts of a protein suitable for forming stable structural motifs and therefore good candidates for immunogenic sites and the production of antibodies.

A large number of computational methods have been used for predicting immunogenic regions of proteins utilising protein structural information, or protein structural properties predicted from sequences.

For example, a tool known in the art as "ElliPro" implements a widely used immunogenic peptide detection method, based upon the identification of protruding regions on protein surfaces. The tool implements a geometric descriptor of ellipticity to characterise the protruding regions (linear and discontinuous). These protrusions are, in theory, complementary to antibody paratope's without conformational change.

Another method, known in the art as "DiscoTope" implements a combination of amino acid features (mainly hydrophobicity), spatial information, and surface exposure. The method may incorporate a spatial neighbourhood definition and half-sphere exposure as surface measure. Hydrophobicity, solvent exposure, and paratope-complementarity are the main features.

A tool known in the art as "Epitopia" is based on a naive Bayes classifier trained on antibody-epitope complex structures. The method may detect patches of 20 amino acids. The method may be used to calculate a relative frequency of amino acids and secondary structures, the relative accessibility, geometry complementary to a CDR, average curvature, and other amino acid scales (mainly hydrophobicity).

Another method is Binding Epitope Prediction from Protein Energetics, known in the art as "BEPPE". BEPPE is based on an energy term derived from MD simulations. BEPPE is based on the idea that recognition sites may correspond to localized regions on the surface whose residues are not optimally stabilized, so that they can tolerate variation in their structure and conformational state.

Other methods known in the art may include Prediction of Antigenic Epitopes on Protein Surfaces by Consensus Scoring (EPCES) and Antigenic Epitopes Prediction with Support Vector Regression (EPVSR). EPCES and EPSVR utilise residue epitope propensity, conservation score, side chain energy score, contact number, surface planarity score, and secondary structure composition.

A tool known as "Spatial Epitope Prediction for Protein Antigens" (SEPPA V3) incorporates glycoprotein-specific features. Generally, SEPPA calculates features for residue triangle patches, using relative accessible surface area, neighbour-based propensity for specific residue occurrence, consolidated and glycosylation-specific AA-indexes, glycosylation ratios for Asn-X-Ser/Thr motifs found in vicinity of triangles, and also takes into account residue spatial clustering. As such, while the immunogenicity scores are per-residue, they are relevant for discontinuous epitope prediction. Sub-models are available based on sub-cellular localization of host-epitope.

A tool known in the art as "BepiPred V2", which is a B-Cell Epitope Predictor, was derived through random forest training of epitope sequences found in 3D epitope-paratope structures. BepiPred V2 incorporates residue volume, hydrophobicity, polarity, solvent accessibility and NetSurfP secondary structure predictions in the model, over a 9 a.a. window.

The above described tools and methods attempt to identify immunogenic regions within proteins, without any specific consideration for peptide stability.

It is therefore desirable to provide a method that is focuses on identifying the most stable peptide sequences within a region that will be most likely to retain similar conformations as peptides compared to the full protein.

It is therefore an aim of at least one embodiment of at least one aspect of the present disclosure to obviate or at least mitigate at least one of the above identified shortcomings of the prior art.

### SUMMARY

The present disclosure provides a method for the provision of peptides that, when isolated from a particular region of a protein, retain one or more of the features of that region. For example, a peptide provided by a method disclosed may retain one or more of the structural, stability and/or conformational features which characterise the region of the full protein from which the peptide has been obtained. In one embodiment, the disclosure provides methods for the provision of peptides which are structurally stable relative to their conformation within a full protein. Use of the term "stable" throughout the ensuing description and claims will be understood by a person skilled in the art to be independent of any flexibility or dynamics that may or may not occur within the full protein. Instead, the term "stable" in the context of the present disclosure refers to peptides that retain a structure and/or confirmation (substantially) resembling the structure and/or confirmation of the corresponding region of the protein.

For convenience, an isolated peptide which retains one or more of the features of the protein region from which it has been isolated, may be referred to as a 'representative peptide' - the peptide being representative (in terms of, for example, structure and/or confirmation) of a particular region of a full protein. Accordingly, the present disclosure provides methods for the provision or identification of, representative peptides.

One of skill will appreciate that such representative peptides have many advantages. In particular, a representative peptide may accurately represent an antigenic or immunogenic region of a protein. As explained in more detail below, such peptides may find particular application in medicine/therapy, as medicaments, in diagnostic/test assays and procedures, in prognostic tests/assays and as vaccine candidates.

Accordingly, the disclosure provides methods for the provision, detection and/or identification of representative peptides (as defined herein).

The disclosure may further provide methods for the provision, detection and/or identification of structurally stable peptides.

As stated, peptides identified by such methods may find application as therapeutic peptides, diagnostic test/procedures, prognostic tests/procedures and/or as antigens for vaccines.

According to a first aspect of the disclosure, there is provided a method of providing a representative peptide, said method comprising:
obtaining a peptide from a protein;
comparing a solvent-accessible surface area (SASA) of the peptide with a SASA of the protein at the corresponding peptide region; and
using a result of the comparison to determine whether or not the peptide is a representative peptide.

In one teaching, a representative peptide is one which, when isolated from a particular region within a protein, retains one or more of the structural, stability and/or conformational features characteristic of that region of the protein.

In another aspect, the disclosure provides a method of identifying structurally stable peptides from a protein, said method comprising:
Obtaining a peptide from a protein;
Comparing a solvent-accessible surface area (SASA) of the peptide with a SASA of a corresponding peptide region within the protein; and
using a result of the comparison to determine whether or not the peptide region is structurally stable relative to the corresponding peptide region within the protein.

A method of this disclosure may focus on identifying peptide sequences most likely to retain structural and/or conformational features which correspond to (or are similar to) the structural and/or conformational features of the protein region from which the peptide is derived. In contrast, prior art methods attempt to identify immunogenic regions within proteins, without specific consideration for peptide stability or the structural and/or confirmation al features of those immunogenic regions when isolated from the protein. That is, the disclosed method seeks to identify regions of a protein (i.e. specific peptide sequences within the protein) which, when isolated from the protein (as a peptide) are likely to retain corresponding, similar or identical structural/conformational features and/or be immunologically similar (to the corresponding protein region) and/or give a corresponding immunogenic signal (corresponding to the immunogenic signal of the protein region).

Advantageously, the disclosed method may provide peptides for use in methods of evaluating or determining the antibody profile of a sample. For example, a peptide provided by a method of this disclosure may be used in an assay to determine whether or not a sample contains or comprises antibodies with specificity for certain antigens, including, for example, viral antigens and/or bacterial antigens. Moreover, a peptide provided by a method of this invention may find application in development of new prognostic and diagnostic assays, as described in more detail below.

Advantageously, given a known immunogenic region within a protein, e.g. as previously identified experimentally, the disclosed methods may facilitate the identification of peptide sequences within and around that region that, when isolated from the full protein, retain the immunogenicity which characterises the protein region.

Advantageously, given a predicted immunogenic region, e.g. identified using any of the computational methods as described in the background section above, the disclosed method may identify specific peptide sequences mostly likely to retain their conformation and be immunologically similar/give an immunogenic signal.

The methods of this disclosure can be contrasted with known peptide tiling strategies, where equally sized peptides may be spaced at regular intervals across a protein sequence and screened. The disclosed method is more efficient, requiring fewer peptides to be tested, and may be more likely to identify peptides that better represent the antigenic properties of the original protein.

A method of this disclosure may be used to select peptides which are representative peptides or structurally stable peptides. Without being bound by theory, a structurally representative or structurally stable peptide may be a peptide having a structure which is similar to (for example substantially similar or identical to) the structure of the corresponding peptide region in the protein. In an example, a representative peptide or structurally stable peptide may be a peptide which retains approximately 60%, 65%, 70%, 75%, 80%, 85%, 90%, 91%, 92%, 93%, 94%, 95%, 96%, 97%, 98%, 98%, 99% or more structural similarity to the corresponding region in the native protein. A degree of structural similarity may correspond to a degree of similarity of a SASA (predicted, simulated, estimated, calculated or measured) of the representative of structurally stable peptide to a SASA of the corresponding region in the native protein.

As stated, peptides of this type may be particular useful as peptides for use in vaccines, diagnostic tests and procedures.

A method of this disclosure may be used to select or identify a representative peptide or structurally stable peptide, wherein a selected peptide may have a SASA similar to the SASA value of the corresponding peptide region in the protein from which the peptide is derived or obtained. A degree of similarity may be in the range of approximately 60%, 65%, 70%, 75%, 80%, 85%, 90%, 91%, 92%, 93%, 94%, 95%, 96%, 97%, 98%, 98%, 99% or more.

A representative peptide identified or provided by a method of this disclosure may be an immunogenic or antigenic peptide. Immunogenic/antigenic peptides generated or provided by a method of this disclosure may exhibit immunogenic and/or antigenic properties which are (substantially) similar or identical to the immunogenic/antigenic properties of the corresponding region within the original protein (i.e. the protein from which the peptide is derived). One of skill will appreciate that a protein may comprise one or more antigenic/immunogenic epitopes. One or more of these epitopes may be clinically significant - that is they form the basis of a diagnostic assay or protective immune response. A method of this disclosure allows a user to identify immunogenic regions of a protein (which regions comprise one or more epitopes), isolate or obtain peptides from those regions and identify those peptides which, despite being isolated from the full protein, retain a chosen epitope. Accordingly, in this context, a peptide with 'similar' immunogenic/antigenic properties will be understood by a skilled person to mean a peptide that:
(a) retains an epitope present in the protein from which the peptide is derived; and/or
(b) retains a functional epitope, wherein a functional epitope is an epitope bound by an antibody which binds to or has specificity or affinity for an epitope present within the full protein; and/or
(c) comprises an epitope/functional epitope present in the protein region from which the peptide is derived;
(d) is bound by an antibody which binds the protein (from which the peptide is derived).

As stated, a peptide which is found (by a method described herein) to have immunogenic/antigenic properties which are (substantially) similar or identical to the immunogenic properties of the corresponding peptide region within the original protein (i.e. the protein from which the peptide is derived), shall be referred to as a representative peptide - the peptide being immunologically representative of the full protein or an immunogenic region thereof.

In one teaching, the comparison step comprises determining the SASA for the peptide and the SASA of the corresponding peptide region within the protein. Any determined difference may then be used to determine whether the peptide is likely to be a representative peptide and/or stable relative to the corresponding region within the protein.

In one teaching, the method may comprise providing two or more, for example a plurality of peptides. A population of peptides for use with a method of this disclosure may be referred as a "test pool" of peptides.

Each peptide of the test pool may be derived from the same or a different protein. Where the peptides are derived from different proteins, each peptide SASA is compared to the SASA value of the corresponding peptide region in the corresponding (and relevant) protein - namely the protein from which the specific peptide has been obtained or derived.

A method of this disclosure may be used to select a peptide or peptides from the test pool, which selected peptide(s) is/are (a) representative peptide(s) or structurally stable.

Additionally, or alternatively, the selected (representative/stable) peptide(s) may be immunologically similar peptide(s).

The selected peptides may each exhibit a SASA value closely representing the SASA value of the corresponding peptide region in the protein from which they have been obtained or derived.

In one teaching, one or more (for example a plurality) of peptides may be obtained from a protein and a SASA value obtained for each peptide and for the corresponding protein region(s). A representative (or stable) peptide (as defined herein) may be the peptide(s) having SASA values which most closely match the SASA value of the corresponding protein region(s). A threshold for the selection of a representative peptide may be set by the user. For example a representative peptide may be the peptide having a SASA value closest to the SASA value of the corresponding protein region. Depending on the number of peptides tested, the user may select peptides with the 2^{nd}, 3^{rd}, 4^{th}, 5^{th}, 6^{th}, 7^{th}, 8^{th}, 9^{th} or 10^{th} closest SASA value match to the corresponding protein region.

It should be understood that the threshold (i.e. level of difference between a SASA of a peptide and a SASA of the corresponding region (ΔSASA)) which denotes a peptide as a representative peptide may vary depending on a number of factors. For example, the user may require peptides which are not necessarily identical (in terms of immunogenicity, conformation and/or structure) to the corresponding region - in which case the threshold (i.e. the magnitude of the SASA difference or ΔSASA) may be larger than if the user requires the selected peptides to be truly representative of the protein regions from which they are derived.

**In** an example, a SASA value closely representing the SASA value of the corresponding peptide region in the protein from which they have been obtained or derived may correspond to the lowest values observed for any peptides within a given region.

A method of selecting a representative or structurally stable peptide from a test pool of peptides may comprise comparing SASA values by determining a difference between the SASA values of each peptide in the test pool with the SASA values of the corresponding peptide region in the protein. The step of comparing SASA values (to determine a difference) may comprise selecting minima from a plot of each difference against a length of each peptide.

The step of comparing SASA values (to determine a difference) may comprise selecting peptides substantially corresponding to minima in the plot of each difference against a length of each peptide, e.g. minima or sufficiently close to minima.

In embodiments, the step of comparing SASA values (to determine a difference) may comprise selecting peptides as close to the minimum as possible, for example within 10% of the minimum to maximum range.

The step of determining the difference may comprise calculating the SASA for each amino acid residue in the context of each peptide and in the context of the (corresponding peptide region in the) protein.

The result of the comparison may be a difference between a size, e.g. a magnitude, of the SASA of the peptide and the SASA of the corresponding peptide region within the protein.

The step of obtaining the peptide from the protein may comprise fragmenting the protein into a plurality of peptides.

The step of obtaining the peptide from the protein may comprise fragmenting the protein into all possible peptides.

For example, for a protein of length *N*, there will be *N*-*L*+1 possible subset peptides of length *L* that can be generated from a full model of the protein. For example, for a 1000-residue protein, there are 991 possible 10mer subset peptides and 901 possible 100mer subset peptides that can be generated.

For each peptide of the plurality of peptides, a SASA of the peptide may be compared with a respective SASA of a corresponding peptide region within the protein.

The method may comprise comparing the results of each comparison to determine whether one or more peptides of the plurality of peptides is likely to be a representative peptide and/or structurally stable relative to the corresponding region in the protein.

Comparing the results may comprise selecting minima from a plot of each result against a length of a respective, corresponding peptide.

Top ranking peptides may be selected (as representative or structurally stable peptides) based upon the entire protein, or can be selected from a specific region of a protein. The disclosed method identifies specific peptides that are most likely to adopt structural conformations, as peptides, that are similar to what may be observed in the full protein. As such, if these regions of the protein bind antibodies in the context of the full protein, then they should also be highly likely to bind the same antibodies when expressed as free or isolated peptides.

In some examples, the protein can be split into a number of regions, and the lowest energy peptide(s) from each region can be selected for experimental testing. Advantageously, the protein structural approach may be used to more efficiently search peptide space, and thus greatly reduce the number of peptides that need to be tested.

In some embodiments, prior knowledge may be utilized to prioritise specific regions of a protein, and use the protein structural approach to select the lowest energy peptides from these regions. This could be based upon previous experimental demonstration of immunogenicity in a specific region, or computational predictions using one or more of the many immunogenicity predictors that have previously been developed.

The step of obtaining the peptide(s) from the protein may comprise selecting the peptide(s) from a specific region of the protein structure based upon a computational prediction of immunogenicity.

The step of obtaining the peptide(s) from the protein may comprise selecting the peptide(s) from a specific region of the protein structure based upon an experimental demonstration of immunogenicity in the specific region.

The protein may correspond to a model of a complete structure of a monomeric protein.

The protein may correspond to a model of a complete structure of a protein as part of a complex.

That is, the protein structure used when calculating the SASA may be the complete structure of a monomeric protein, or it could be the complete structure of a protein as part of a complex. This may depend on what is believed to be the most biologically relevant context for the protein.

The method may comprise a preceding step of selecting the protein from a plurality of proteins in a database.

The step of selecting the protein may comprise selecting a protein structure computationally predicted from its amino acid sequence.

The step of selecting the protein may comprise selecting an experimentally determined protein structure, wherein the structure has been experimentally determined by one of: X-ray crystallography; nuclear magnetic resonance spectroscopy; or cryo-electron microscopy.

The experimentally determined protein structure may be selected based on the protein being determined to be present as part of a biologically relevant homomeric or heteromeric complex.

The experimentally determined protein structure may be selected based on a fraction of a full-length of the protein that is present.

The experimentally determined protein structure may be selected based on at least a portion of the protein being provided with an atomic resolution.

The step of obtaining the peptide(s) from a protein may comprise generating a model of a structure of the/each peptide by extracting amino acid residues of the protein.

The term "peptide" will be understood to refer to a continuous subset of the full-length protein structure. A structure of the peptide can be generated by extracting the residues.

The step of obtaining the peptide(s) from a protein may comprise fragmenting the protein into peptides comprising between 10 and 100 amino acids.

A method of this disclosure may be used to identify structurally stable parts of an antigen.

In the context of this disclosure, an antigen may comprise any protein or peptide which might raise an immune response in a host. The term 'antigen' may include proteins or peptides of microbial origin (for example bacterial and/or viral proteins) and/or, for example, tumour (or other self) antigens and the like.

In one teaching, a method of this disclosure may be used to identify representative and/or structurally stable regions of a viral or bacterial antigen. Without wishing to be bound by theory, the identification of representative and/or structurally stable regions within an antigen may lead to the provision of peptides from those regions with therapeutic, diagnostic, prognostic and/or vaccine use. Furthermore, for the avoidance of doubt a peptide which is representative of a viral or bacterial antigen is a peptide which retains an epitope or some aspect of the structural, conformational or antigenic properties of the viral/bacterial antigen.

It should be noted that a method of this disclosure may be applied to any antigen, irrespective of its source (self, tumour, viral, bacterial etc.). By way of example only, a method of this disclosure may use peptides (for example peptide antigens) derived from, for example a bacterial antigen, a viral antigen, an influenza antigen, a coronavirus antigen, an EBV antigen or a tumour antigen.

In one teaching, the protein may comprise an EBV antigen - for example an EBV gB protein (as encoded by the BALF4 gene), an EBV capsid protein p18 as encoded by the BFRF3 gene), an EBV EBNA protein (as encoded by any of the EBNA1, EBNA3B, EBNA2, EBNA3A, EBNA3B or EBNALP genes), an EBV gp60 protein (as encoded by the BILF1 gene), an EBV capsid protein p23 (as encoded the BLRF2 gene), an EBV tegument protein (as encoded by any of the BBLF1, BRRF2 genes), an EBV latent membrane protein 1 (as encoded by the LMP1_2 gene).

In one teaching, the protein may comprise a Coronavirus antigen. The coronavirus antigen may be selected from the group:
(i) a SARS antigen;
(ii) a SARS-CoV-2 antigen (including any SARS-CoV-2 antigen from any SARS-CoV-2 strain/variant);
(iii) a SARS-CoV-2 Alpha (WHO label) antigen
(iv) a SARS-CoV-2 Beta (WHO label) antigen
(v) a SARS-CoV-2 Gamma (WHO label) antigen
(vi) a SARS-CoV-2 Delta (WHO label) antigen
(vii) a SARS-CoV-2 Omicron (WHO label) antigen
(viii) a MERS antigen;
(ix) a Nucleocapsid (N) protein;
(x) a Membrane (M) protein;
(xi) a Spike (S) protein;
(xii) an Envelop (E) protein; and
(xiii) any fragment of any of (i)-(xii)

It should be noted that as used herein, the term SARS-CoV-2 includes any of the recognised strains/variants. As such a reference to a SARS-CoV-2 S-protein would embrace any protein (peptide or antigen) from any of the SARS-CoV-2 strains/variants. Likewise, a reference to a SARS-CoV-2 S protein would embrace any S protein (or fragment thereof) from any SARS-CoV-2 variant/strain.

In one teaching, the protein may be a SARS-CoV-2 S-protein.

In another teaching the protein may be a SARS-CoV-2 N protein.

According to a further aspect of the disclosure, there is provided a use of the method of according to the first aspect to identify representative or structurally stable peptides within a SARS-CoV-2 protein.

The SARS-CoV-2 protein may be the SARS-CoV-2 S protein.

The SARS-CoV-2 protein may be the SARS-CoV-2 N protein.

According to a further aspect of the disclosure, there is provided a representative peptide or a stable peptide identified or obtainable by a method of this disclosure.

According to a further aspect of the disclosure, there is provided a method of peptide synthesis comprising: identifying one or more representative and/or stable peptides using a method of this disclosure; synthesising DNA sequences corresponding to the one or more peptides; and cloning the DNA sequences into expression vectors.

The expression vectors may be for mammalian and/or bacterial cells.

The method may comprise a subsequent step of transfecting the expression vectors into cells (for example Expi293 cells) for (mammalian) protein expression.

The method may comprise a subsequent step of transforming the expression vectors into a bacterial cell, for example a T7Express *E.coli* cell.

According to a further aspect of the disclosure, there is provided a method of peptide prioritisation comprising the identification of putatively informative peptides from a plurality of peptides corresponding to structurally stable motifs identified in a protein according to the method of the first aspect. The identification step may be achieved using, for example, an enzyme-linked immunosorbent assay (ELISA).

According to a further aspect of the disclosure, there is provided a computer program comprising instructions which, when the program is executed by a computer, cause the computer to carry out the steps of the method of the first aspect.

The computer program may be configured to calculate the SASA of the peptide and the SASA of a corresponding peptide region within the protein, wherein: the SASA is defined as a locus of the centre of a probe sphere as it rolls over the Van der Waals surface of the amino acid residue; and surface points are generated on an extended sphere about each atom of the model of the amino acid residue, at a distance from the atom centre equal to the sum of the atom and probe radii, and eliminating those points that lie within equivalent spheres associated with neighbouring atoms are eliminated.

According to an aspect of the disclosure, there is provided a system comprising a processor and a memory including the computer program, and configured to execute the computer program to carry out the steps of the method according to the first aspect.

The result of the comparison, e.g. a difference between the SASA of the peptide and the SASA of the corresponding peptide region within the protein, may correspond to a pseudo-energy term that may be effectively used to score all of the peptides. Peptides exhibiting lower ΔSASA values form fewer intramolecular or intermolecular contacts outside of the peptide region, which means their conformation when expressed as a peptide may be more likely to resemble their conformation within the context of the full protein structure.

ΔSASA values may scale with peptide length, simply because longer peptides have more amino acid residues. While this may not affect comparisons between peptides of the same length, this may mean that ΔSASA values are not necessarily directly comparable between peptides of different lengths. Therefore, in example embodiments peptides may be scored using ΔSASA per residue, i.e. ΔSASA divided by peptide length.

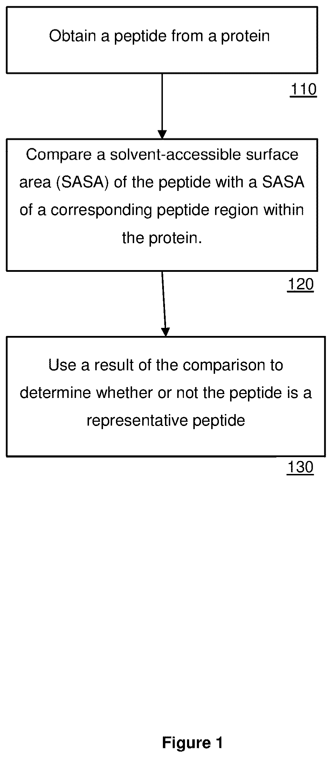

Furthermore, even when normalising ΔSASA, there may still be some correlation with protein length. Therefore, to select optimal peptides of varying lengths, ΔSASA may be plotted against peptide length, and local minima can be selected, as described in more detail below with reference to Figure 1, which depicts a graph of peptide energy versus length.

Top peptides may be selected based upon the entire protein, or may be selected from a specific protein region based upon prior knowledge, e.g. previous experimental demonstration or computational prediction of immunogenicity.

According to a further aspect of the disclosure, there is provided a use of the method according to this disclosure to identify stable or representative epitopes of an antigen. As stated, a stable or representative epitope may be one which retains the ability to bind an antibody when presented in an isolated peptide and within the context of the full protein from which the peptide is derived.

As stated above, the antigen may be from any source.

In one teaching, the antigen is a SARS-CoV-2 antigen, for example an S-protein or part thereof) and/or an N-protein (or part thereof).

Advantageously, any of the disclosed methods may be used to derive immunogenic/antigenic peptides that may have considerable use in diagnostic and prognostic methods, antibody detection and profiling, e.g. 'fingerprinting', vaccine development and variant testing.

By way of example, the disclosed methods may be used to derive SARS-CoV-2 immunogenic/antigenic peptides that may have considerable use in diagnostic and prognostic methods, antibody detection and profiling, e.g. 'fingerprinting', vaccine development and variant testing.

The present disclosure provides peptides having the sequences represented by SEQ ID NOS: 1-17 and 30-49.

One or more of the peptides described herein - especially those provided as SEQ ID NOS: 1-17 and 30-49, may be joined together to form a larger peptide. These peptides may be referred to as 'daisy-chained' peptides - that is they comprise two or more shorter peptides (selected from the cohort of peptides presented as SEQ ID NOS: 1-17 and 30-49) linked by one or more linkers.

Any of the peptides described herein (including those provided by SEQ ID NOS: 1-17 and 30-49) may be joined or linked to another using a linker molecule. A linker molecule may comprise a peptide of any suitable length. One of skill would be familiar with an array of suitable linker molecules including the sequence of suitable peptide linker candidates. Nevertheless, by way of example suitable peptide linkers may include those provided by SEQ ID NOS: 18 and 19 below:
In view of the above, this disclosure further provides the following peptides - each of which represents an example of a 'daisy-chained' peptide (each one of SEQ ID NOS: 20-25 comprising a number of the peptides given as SEQ ID NOS: 1-17 joined by a linker (such as a linker provided by any of SEQ ID NOS: 18 or 19)).

As stated, the peptides disclosed herein (including those provided by SEQ ID NOS: 1-17 and 20-29 and 30-49 have a variety of uses.

Accordingly, the disclosure provides any one of SEQ ID NOS: 1-17 and 20-29 and 30-49 for use in medicine.

Also disclosed is any one of SEQ ID NOS: 1-17 and 20-29 and 30-49 for use in a method of fingerprinting or profiling an antibody response. For example a series of peptides - selected by a method of this disclosure to be representative of a particular antigen or antigens, may be used as the basis of a test to determine the antibody profile (or fingerprint) of a particular sample. That sample may be provided by or obtained from a subject thought to be infected with a pathogen expressing the antigen or from a subject infected with or convalescing from an infection. Antibody fingerprinting or profiling information may be used to stage an infection and/or to determine a subjects immune status. The profiled response may be an anti-viral response, an anti-SARS-CoV-2 response or an anti-EBV response.

The disclosure further provides any one of SEQ ID NOS: 1-17 and 20-29 and 30-49 for use in a method of diagnosis. The diagnostic method may provide a viral diagnosis, a SARS-CoV-2 diagnosis or an EBV diagnosis (the precise peptide or peptides selected for use depending on the disease or condition to be diagnosed - for example, a method for use in diagnosing a disease or condition associated with SARS-CoV-2, may use any one or more of the SARS-CoV-2 proteins described herein).

The disclosure also provides any one of SEQ ID NOS: 1-16 and 20-29 and 30-49 for use in a vaccine. The vaccine may be an anti-SARS-CoV-2 vaccine or an anti-EBV vaccine (the precise peptide or peptides selected for use depending on the purpose of the vaccine - for example a vaccine for use in raising an immune response against SARS-CoV-2 may use any one or more of the SARS-CoV-2 proteins described herein).

Also disclosed is a method of detecting an antibody response, said method comprising probing a sample for the presence of antibodies which bind to or which have specificity/affinity for a protein or peptide comprising (or consisting/consisting essentially of) any one or more of SEQ ID NOS: 1-17, SEQ ID NOS: 20-29 or SEQ ID NOS: 30-49. The antibody response may be an anti-SARS-CoV-2 antibody response or an anti-EBV antibody response. As above, the precise peptide or peptides selected for use in any method of detecting an antibody response disclosed herein will depend on the specificity of the target antibody; for example, a method of detecting an anti-SARS-CoV-2 antibody response may use any one or more of the SARS-CoV-2 proteins described herein.

The disclosure provides the use of any one of SEQ ID NOS: 1-17 and SEQ ID NOS: 20-29 or SEQ ID NOS 30-39 in a method of detecting anti-SARS-CoV-2 antibodies in a sample.

The disclosure provides the use of any one (or more) of SEQ ID NOS: 30, 31, 32, 33, 34, 35, 36, 37, 38, 39, 40, 41, 42, 43, 44 or 45 in a method of detecting anti-EBV antibodies in a sample.

The methods described herein may be *in vitro* methods.

A method of detecting an antibody response may be used to detect antibodies of any isotype, including for example IgG, IgA, IgM, IgD and/or IgE antibodies.

In one teaching, the disclosure provides a method of detecting an antibody response, which response is the result of a (natural) infection or vaccination, said method comprising probing a sample for the presence of antibodies which bind to or which have specificity/affinity for a protein or peptide comprising (or consisting/consisting essentially of) any one or more of SEQ ID NOS: 20, 23, 24, 25, 27, 28 and/or 29. The detected response may be an anti-SARS-CoV-2 response.

In a further teaching, the disclosure also provides a method of detecting an antibody response, which response is the result of a (natural) infection but not vaccination, said method comprising probing a sample for the presence of antibodies which bind to or which have specificity/affinity for a protein or peptide comprising (or consisting/consisting essentially of) any one or more of SEQ ID NOS: 21, 22 and/or 26. The response may be an anti-SARS-CoV-2 response.

The disclosure also provides a method of detecting an anti-SARS-CoV-2 spike antibody response, which response is the result of a (natural) infection or a vaccination, said method comprising probing a sample for the presence of antibodies which bind to or which have specificity/affinity for a protein or peptide comprising (or consisting/consisting essentially of) any one or more of SEQ ID NOS: 25 or 29.

Disclosed herein is a method of identifying a sample which may contain influenzaA antibodies (i.e. antibodies with an ability to bind or with specificity/affinity for an influenzaA antigen) with cross-reactivity to a SARS-CoV-2 antigen. Said method comprising probing a sample for the presence of antibodies which bind to or which have affinity/specificity for a peptide or protein comprising (or consisting/consisting essentially of) SEQ ID NO: 17. A sample which contains antibodies which bind to SEQ ID NO: 17, may also contain antibodies which cross react with a SARS-CoV-2 antigen. The results of this assay may be important as a diagnostic test for SARS-CoV-2 antibodies may comprise a peptide having the sequence of SEQ ID NO: 17 - this peptide may cross react with anti-influenzaA antibodies and this may lead to false positive results.

Within the context of these methods, the term 'sample' may be any type of sample likely to contain antibodies (of any isotyope). The sample may comprise a biological fluid such as blood (whole blood or any fraction thereof (including, for example, serum), saliva or mucosal fluids. The sample may comprise a tissue sample, a biopsy, a wash and/or a scraping.

Also disclosed is a method of making an antibody, said method comprising raising an immune response using a peptide identified by a method of this disclosure. In a method of this type, the peptide may be a peptide which is representative of a particular antigen. In other words, the peptide may retain one or more of the epitopes present in the antigen. Those epitopes may remain functional in the peptide.

The disclosure may provide a method of making a monoclonal antibody, said method comprising introducing either:
a peptide of this disclosure; or
a peptide prepared by a method of this disclosure;
to a mouse and forming a hybridoma. The method may further comprise the step of culturing the hybridoma under conditions which induce the expression or production of monoclonal antibodies which bind to or which have affinity and/or specificity for the peptide.

The disclosure also provides a method of monitoring a response to therapy. A method of this type may comprise using peptides generated, obtained or obtainable by a method of this disclosure to profile the antibody response in a sample or series of samples. The sample(s) may be obtained from a subject being treated for a particular disease. The peptides may each be representative of a protein or antigen expressed by a pathogen associated with the disease. A series of samples may be obtained or provided by a subject at different times during the subjects treatment regime - for example before, during and/or after treatment. By profiling the antibody response in each sample, it may be possible to determine the success of a particular treatment. For example, where a treatment helps resolve an infection, this may reflect in an increase or decrease of certain antibodies in the sample. A method of this disclosure may provide peptides which are fully representative of an antigen expressed by the relevant pathogen and as such, a method according to this embodiment represents an efficient and accurate method of monitoring a response to a therapy.

In another teaching, the disclosure provides a method of detecting an immune response to a microbial, viral or bacterial variant. Such methods may rely on peptides obtained or selected (via a method of this disclosure) from some antigen characterising the microbial, viral or bacterial variant. For example, a method of detecting an immune response to a variant pathogen (for example a variant SARS-CoV-2 (e.g. omicron)), may use a method of this disclosure to provide or select peptides which are representative of all or part of a variant antigen (for example a variant SARS-CoV-2 spike protein) expressed by the variant pathogen. A method may further comprise the step of detecting in a sample (for example a sample of blood or a fraction thereof) an antibody binding to one or more of the selected representative peptide(s). A sample may be contacted with a representative peptide under conditions which permit binding between any antibodies present in the sample and the peptide(s). The detection of antibodies binding to the representative peptides, indicate that the sample was provided by or obtained from a subject infected with the variant pathogen.The above summary is intended to be merely exemplary and non-limiting. The disclosure includes one or more corresponding aspects, embodiments or features in isolation or in various combinations whether or not specifically stated (including claimed) in that combination or in isolation. It should be understood that features defined above in accordance with any aspect of the present disclosure or below relating to any specific embodiment of the disclosure may be utilized, either alone or in combination with any other defined feature, in any other aspect or embodiment or to form a further aspect or embodiment of the disclosure.

### BRIEF DESCRIPTION OF THE DRAWINGS

These and other aspects of the present disclosure will now be described, by way of example only, with reference to the accompanying drawings and Sequence IDs, wherein:
- **Figure 1**: depicts a method of identifying stable peptides in a protein, according to an embodiment of the disclosure;
- **Figure 2**: depicts another method of identifying stable peptides in a protein, according to a further embodiment of the disclosure;
- **Figure 3**: depicts an example of characterisation of predicted stability of SARS-CoV-2 spike peptides
- **Figure 4**: depicts SARS-CoV-2 and similarity to other beta-coronaviruses;
- **Figure 5**: depicts an inverse correlation between surface area and protein stability (buried residues are coloured in blue, solvent accessible residues are marked in red);
- **Figure 6**: depicts peptide length selected for SARS-CoV-2 proteins;
- **Figure 7**: depicts selection of peptides across SARS-CoV-2 spike and nucleocapsid proteins;
- **Figure 8**: depicts a visual representation of immunogenic peptide identification for SARS-CoV-2 nucleoprotein;
- **Figure 9**: depicts a visual representation of immunogenic peptide identification for SARS-CoV-2 spike protein;
- **Figure 10**: depicts the surface spike protein decorated with a vast array of N-linked glycosylation sites;
- **Figure 11**: depicts design of mammalian and bacterial protein expression constructs;
- **Figure 12**: depicts high throughput cloning of peptide library utilising technology from synthetic biology;
- **Figure 13**: depicts an approach for protein purification and ELISA assay, wherein His-tagged proteins were purified using a KingFisher robot using Ni-NTA magnetic agarose beads;
- **Figure 14**: depicts ELISA used to identify immuno-reactive peptides to patient serum, wherein patient serum/plasma was either pooled serum from individuals infected with coronavirus (POS), pooled serum from individuals that were not exposed to coronavirus (NEG) or vaccinated individuals (VAX);
- **Figure 15**: depicts a Ratio of ELISA signal between positive (convalescent) and negative (pre-2019) pooled sera for the nucleocapsid and spike proteins;
- **Figure 16**: depicts differences in reactivity between peptides purified from bacteria and mammalian cells, wherein these are likely due to post-translational modifications;
- **Figure 17**: shows that different peptides can discriminate between SARS-CoV-2 variants;
- **Figure 18**: depicts a heat map showing individual positive (y-axis, S1-S23) or negative sera (y-axis, S24-S37 ) reactivity (low, blue; cream, high) to peptides (x-axis), wherein significant heterogeneous reactivity is observed; and
- **Figure 19**: depicts prioritization of 5 peptides used in an ELISA assay to discriminate between positive and negative patient samples. Results show high sensitivity and specificity.

### DETAILED DESCRIPTION OF EMBODIMENTS

Figure 1 depicts a method of identifying stable peptides in a protein, according to an embodiment of the disclosure.

In a first step 110, a peptide is obtained from a protein. This may, for example, comprise the generation of a model of a structure of a peptide by extracting amino acid residues of the protein. In embodiments, a plurality of peptides may be obtained from the protein. In some embodiment, as many as all possible peptides may be obtained from the protein. In embodiments, the obtained peptide(s) may comprise between 10 and 100 amino acids.

In a second step 120, the solvent-accessible surface area (SASA) of the peptide is compared with the SASA of a corresponding peptide region within the protein.

The/each SASA may be calculated using a known means. For example, the SASA may be defined as a locus of the centre of a probe sphere as it rolls over the Van der Waals surface of the amino acid residue. Known tools may calculate a SASA based on a "Shrake-Ruply" algorithm, or the like. For example, tools may calculate a SASA by generating surface points on an extended sphere about each atom of the model of the amino acid residue, at a distance from the atom centre equal to the sum of the atom and probe radii, and eliminating those points that lie within equivalent spheres associated with neighbouring atoms are eliminated.

In other examples, known tools may calculate a SASA based on approximating atomic surfaces from Linear Combinations of Pairwise Overlaps of spheres, in a method known in the art as "LCPO".

The comparison may comprises determining a difference between a size of the SASA of the peptide and the SASA of the corresponding peptide region within the protein

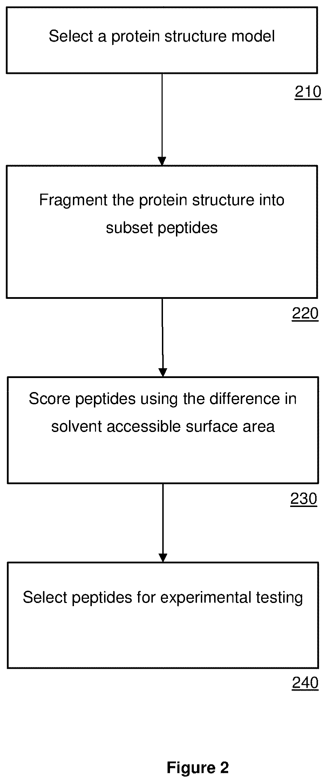

In a third step 130, a result of the comparison is used to determine whether or not the peptide is likely to be stable relative to the corresponding region in the protein. Furthermore, as described in more detail below with reference to the example method of Figure 2, the result of the comparison may be compared to a further result of the comparison of one or more further peptides to determine which peptide region is most likely to form a stable peptide.

Figure 2 depicts a method of identifying stable peptides from a protein, according to a further embodiment of the disclosure.

In a first step 210, a protein structure model is selected. The protein structure may be a structural model of a protein in the form of a Protein Data Bank (PDB) file, which holds the three-dimensional co-ordinates of all atoms in the model.

In some embodiments, an experimentally determined model, e.g. with X-ray crystallography, nuclear magnetic resonance spectroscopy, or cryo-electron microscopy, may be selected.

Various criteria may be used to select a most appropriate experimentally determined protein structure for the system of interest, with a goal of selecting a model that most closely resembles what the protein is likely to look like when encountered by human antibodies.

For example, if the protein of interest is likely to be present as part of a biologically relevant homomeric or heteromeric complex, then a model of the complex may be selected. Other factors such as a fraction of the full-length protein present and atomic resolution may also be considered when selecting the best available structure model.

In some example embodiments, such as where no experimentally determined structure model is available or suitable for the protein of interest, a computationally predicted model may be used.

One or more known models for computationally prediction may be employed. An example of a known method for computationally predicting the three-dimensional structure of a protein is described in "Highly Accurate Protein Structure Prediction with AlphaFold", John Jumper, Richard Evans, et al. Nature 2021.

In a second step 220, the protein structure is fragmented into subset peptides. In some example embodiments, the protein structure is fragmented into all possible subset peptides.

The term "subset peptide" will be understood by a person of skill in the art to refer to a continuous set of residues from the full-length protein structure. In an example embodiment, the structure of a peptide may be generated by extracting specific amino acid residues from the full PDB file into a new smaller PDB file.

In some embodiments, peptides of any length may be used.

In some embodiments, a range of lengths of the peptides may be restricted to peptides having approximately 10 to 100 amino acid residues in length.

In an example, for a protein structure of length N, there will be N-L+1 possible subset peptides of length L that can be generated from the full PDB file. For example, for a 1000-residue protein, there are 991 possible 10mer subset peptides and 901 possible 100mer subset peptides that can be generated.

In a third step 230, the peptides are scored using the difference in solvent accessible surface area.

The SASA for each amino acid residue from each subset peptide, both within the context of the isolated peptide, and within the context of the full protein structure is calculated. The "isolated peptide" is the PDB file representing the structure of the subset peptide by itself. The "full protein structure" is the PDB file representing the full structure of interest, e.g. as selected in the first step 110.

The SASA may be calculated using a known means. For example, the SASA may be calculated as described above with reference to the method of Figure 1.

The difference between the two SASA values (ΔSASA = SASA_{isolated} - SASA_{full}) is used as a pseudo-energy term to score all of the peptides. Peptides with lower ΔSASA values form fewer molecular contacts outside of the peptide region; thus fewer contacts will be disrupted when expressed as a peptide, and peptide conformation is more likely to resemble the full protein structure.

In a fourth step 240, peptides are selected for experimental testing. To find the top ranking peptides over a range of lengths, ΔSASA can be plotted against peptide length. Through examination of this plot, local minima (or peptides sufficiently close to local minima) can be selected that represent the lowest energy peptides of different lengths.

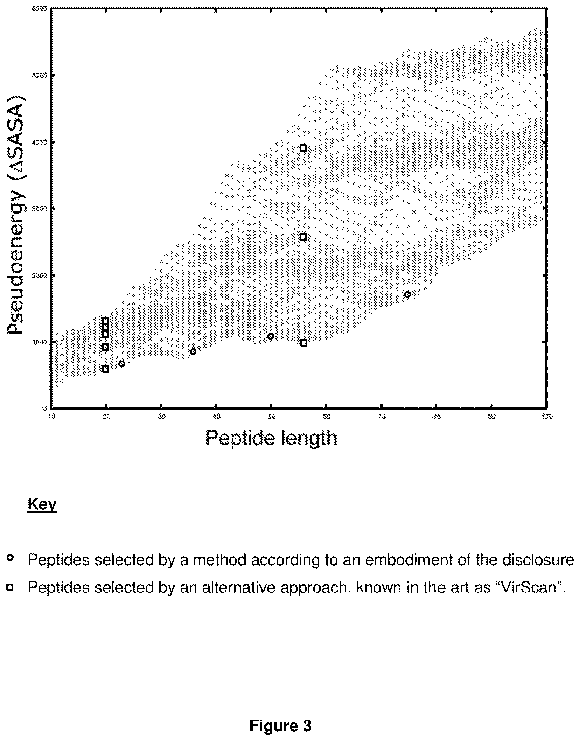

Figure 3 depicts an example of a thermodynamic characterisation of SARS-CoV-2 spike peptides. In Figure 3, peptides optimally identified using the methods disclosed herein are highlighted. For comparison, peptides identified using an alternative approach, known in the art as "VirScan", are also depicted. It can be seen that the pseudo-energy scores associated with peptides identified using the alternative approach are substantially different from pseudo-energy scores associated with peptides selected using the methods disclosed herein.

In embodiments, top ranking peptides may be selected based upon the entire protein, or may be selected from a specific region of a protein. The disclosed method may identifies specific peptides that are most likely to adopt structural conformations, as peptides, that are similar to what may be observed in the full protein. If these regions of the protein bind antibodies in the context of the full protein, then it may be assumed that the regions of the protein are highly likely to bind the same antibodies when expressed as subset peptides.

The disclosed method may be used in at least two ways.

First, the protein can be split into a number of regions, and the lowest energy peptide(s) from each region can be selected for experimental testing. Advantageously, the disclosed method efficiently searches a peptide space, thus greatly reduce a number of peptides that may need to be tested.

Second, prior knowledge of a person skilled in the art may be used to prioritise specific regions of a protein, and the disclosed method may be used to select lowest energy peptides from such specific regions. For example, the prior knowledge may be based upon previous experimental demonstration of immunogenicity in a specific region, or computational predictions using one or more of the many immunogenicity predictors that have previously been developed.

Proteins have complex three dimensional structures and surface exposed amino acids that, when injected into an animal, e.g. human, mouse, rabbit, may trigger an immune response, resulting in the generation of antibodies to specific protein epitopes.

This disclosure relates to a thermodynamic prediction method for identifying which parts of a protein can yield representative peptides and/or peptides which are can structurally stable. Such peptides are good candidates for immunogenic sites and the production of antibodies. Further described is a subsequent prioritisation of informative peptides.

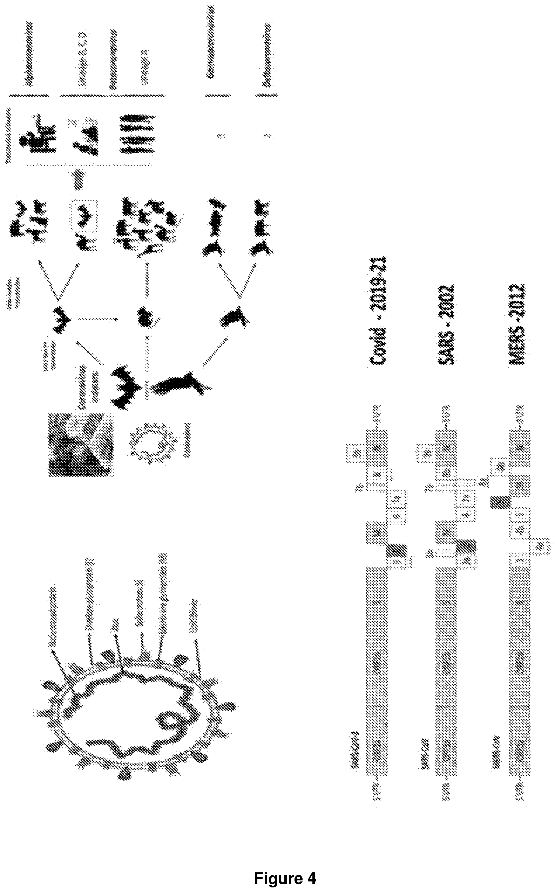

In an example of the utility of the disclosed methods, two hundred different peptides were synthesised, selected from SARS-CoV-2, in mammalian and bacterial cells using novel expression vectors where the viral peptides were fused to stabilising proteins and attached to a purification tag. See, for example, Figure 4, which depicts SARS-CoV-2 and similarity to other beta-cornaviruses.

In the example, proteins were synthesised in an appropriate host and purified. In the example, purified fusion proteins from SARS-CoV-2 were then used in an ELISA assay to show reactivity to patient serum. Patient serum/plasma was either pooled from individuals infected with coronavirus, pooled serum from individuals that were not exposed to coronavirus or individual samples from positive, negative or vaccinated individuals. From this screen, individual immunogenic peptides were prioritised for further study.

As descried above, this disclosure may be useful for evaluating the antibody repertoire to proteins, viruses, bacteria or other immunogenic species. Specifically, the disclosed methods when combined with the prioritisation of specific peptides may provide a useful approach for the development of new prognostic and diagnostic assays.

The disclosed methods relate to identification of peptide sequences that would be most likely to adopt similar conformations when synthesised as peptides compared to their context within the full-length protein or protein complex. In an example, for a 1000 residue protein there are 95050 possible sub-peptides between 10 and 100 amino acids in length. It may be desirable to find those most likely to illicit an immunogenic signal. However, it may be time consuming to screen this many peptides using complex energy functions. As such, the disclosed method relates to a property that is relatively simple to compute from 3D protein structures and is directly related to the energy of protein folding: the solvent-accessible surface area.

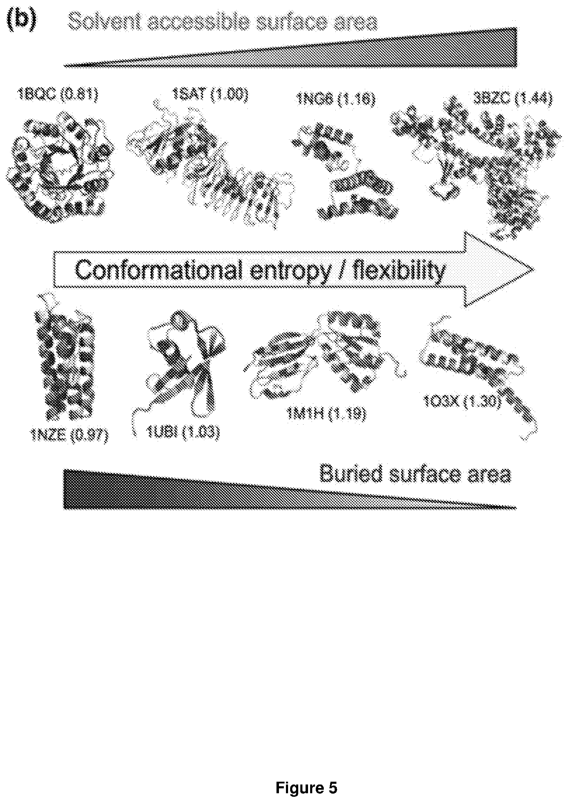

Solvent-accessible surface area may be useful for predicting protein stability, flexibility and assembly, and may be competitive with much more computationally intensive computational modelling strategies. See for example Figure 5, which depicts an inverse correlation between surface area and protein stability, wherein buried residues and solvent accessible residues are identified.

In an example, to identify thermodynamically stable peptides the protein is broken into small fragments and the difference in solvent-accessible surface area between the free peptide, and the peptide region within the context of the full structure/complex are compared. See for example Figure 3 which depicts thermodynamic characterisation of SARS-CoV-2 spike peptides, wherein optimally identified peptides and peptides identified from alternate VirScan approach are identified.

From this, specific candidate peptides may be identified in a non-obvious manner. Top-ranking peptides may be either directly selected for experimental characterisation, or further screened computationally using more complex energy functions and molecular modelling.

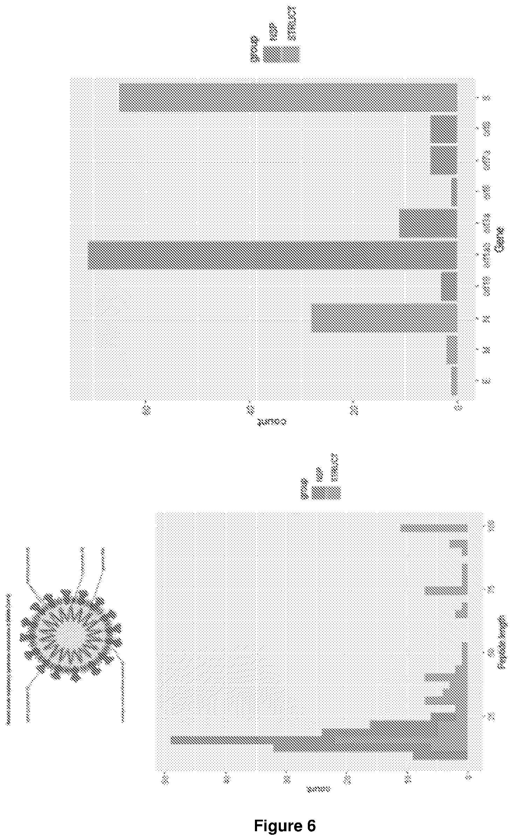

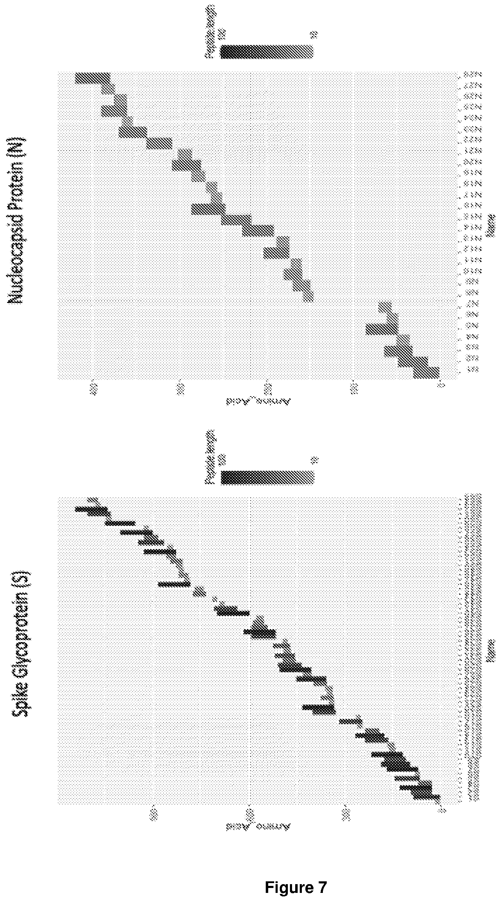

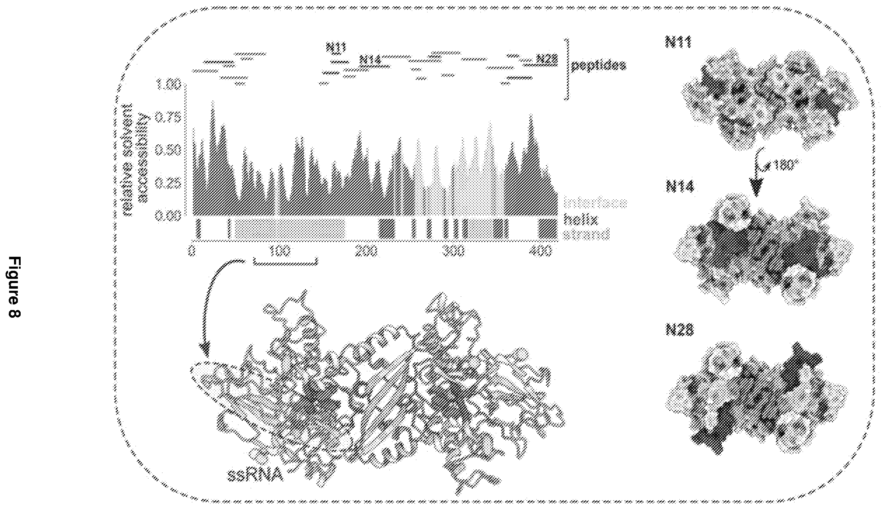

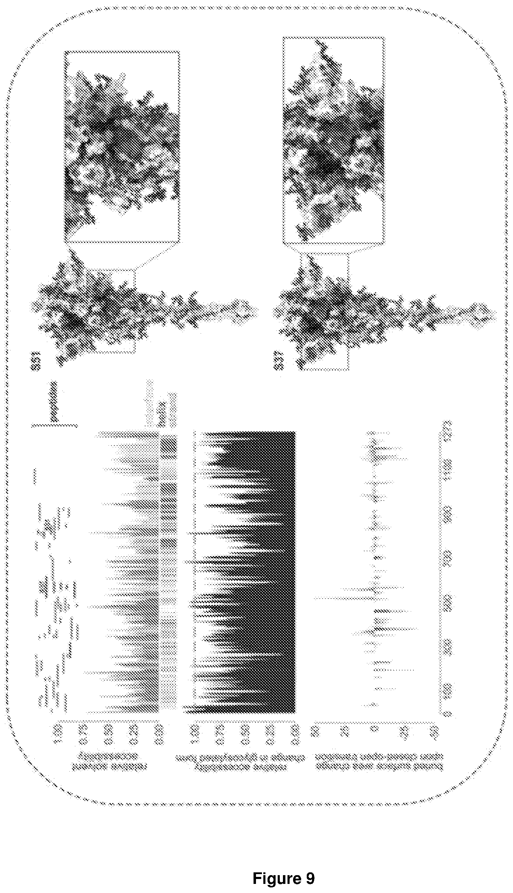

The disclosed methods may be exploited to identify stable potentially immunogenic epitopes of SARS-CoV-2, with a focus on short peptides. See for example Figures 3 and 6 to 9. Additional thermodynamic and functional information may be added to this pipeline such that final peptide selection may be based on a combination of both energy parameters and other protein characteristics.

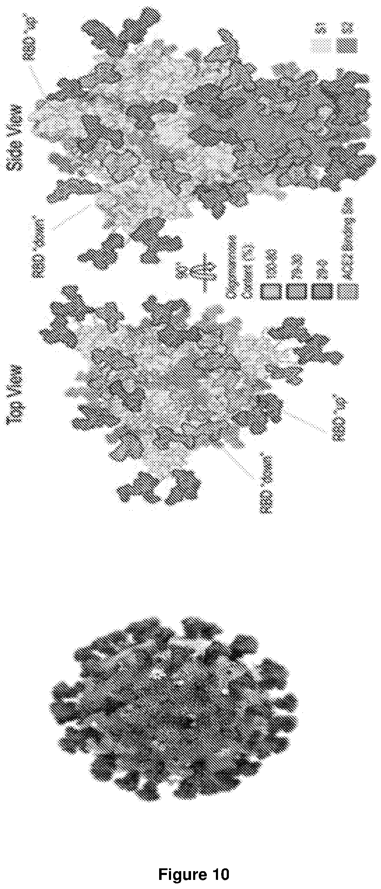

For example, individual peptides that have protein modifications may be further prioritised. See Figure 10, for example.

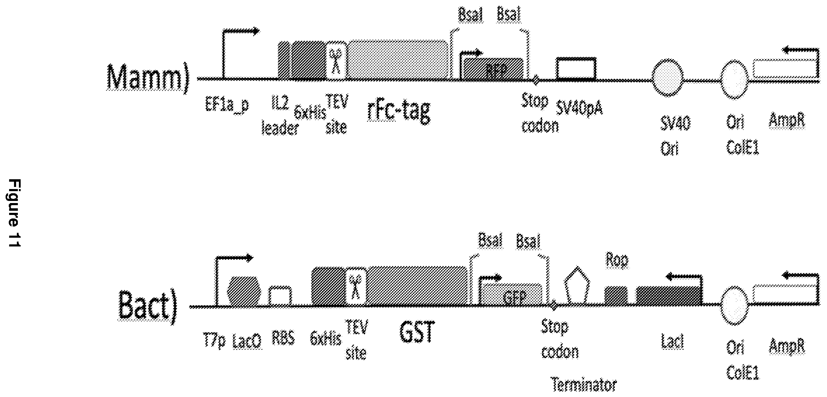

After identification of putative immunogenic peptides, DNA sequences corresponding to the fragments may be synthesised with directional Bsal restriction enzyme sites. DNA fragments may then be cloned into expression vectors. See, for example, Figure 11.

In an example, new vectors may be designed to include useful characteristics to enable stable high level protein expression. In the described example, in terms of construct design there were two flavours (Figure 11):
(i) a vector for expression in mammalian cells; and
(ii) a vector for expression in bacterial cells.

In the described example, for both mammalian and bacterial cells DNA libraries could be efficiently ligated into vectors using standard molecular cloning techniques .

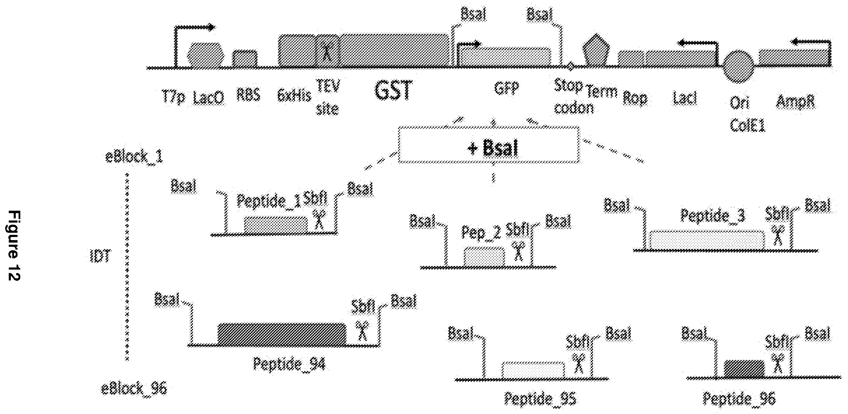

In the described example, the shared components of the vectors are cell type specific promoter, histidine purification tag, fusion protein domain, high throughput cloning site, termination site. The bacterial construct has a GST fusion protein domain, whilst the mammalian construct has a rabbit Fc fusion domain. During the project, different constructs were synthesised to identify those that had the best and most consistent protein expression. DNA libraries were cloned into vectors as described (see Figure 12) and individual clones were characterised and sequenced.

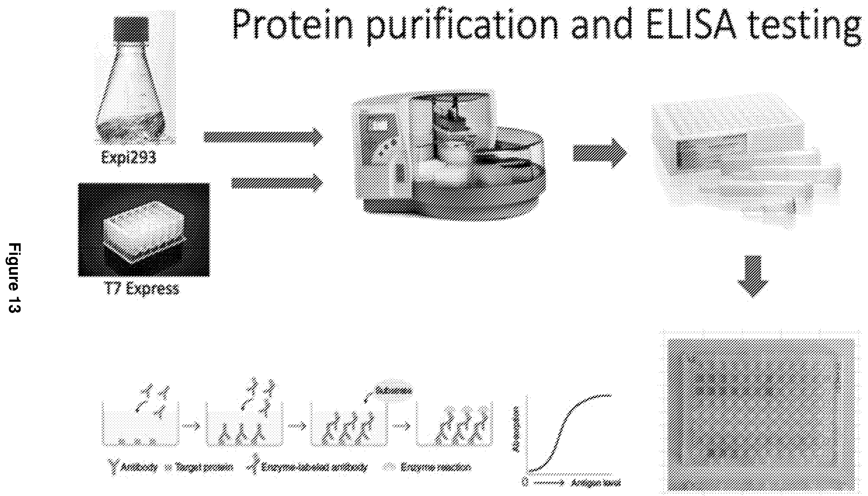

In the described example, after cloning, vectors were transfected into Expi293 cells for mammalian protein expression or transformed into T7Express E.coli cells and standard approaches were used for protein expression, See Figure 13, for example.
- Bacteria: Transformed cells were grown to late log phase, subcultured and grown to an OD of 0.4, shifted to 18C and then IPTG added to 0.5 uM. After 24 h growth cells were harvested, lysed and the protein of interest purified using magnetic nickel agarose beads.
- Mammalian: Transfected cells were grown for 5 days. Supernatant was harvested and the protein of interest purified using magnetic nickel agarose beads

In the described example, after purification proteins were desalted, quantified and stored in 10% glycerol in TEP buffer.

In the described example, an ELISA assay was used to identify putatively informative peptides. See Figure 13. Purified peptides were coated onto an ELISA plate at a defined concentration. Positive or negative serum was added to the plate and binding was monitored using a standard colorimetric assay.

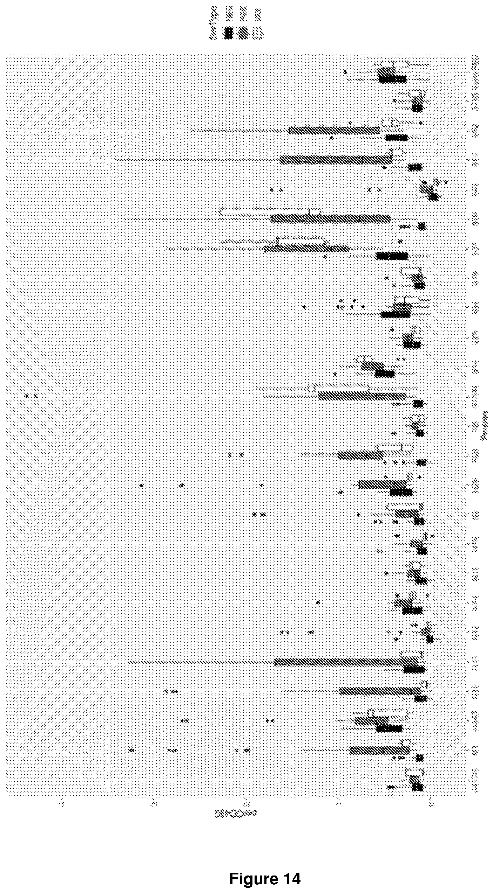

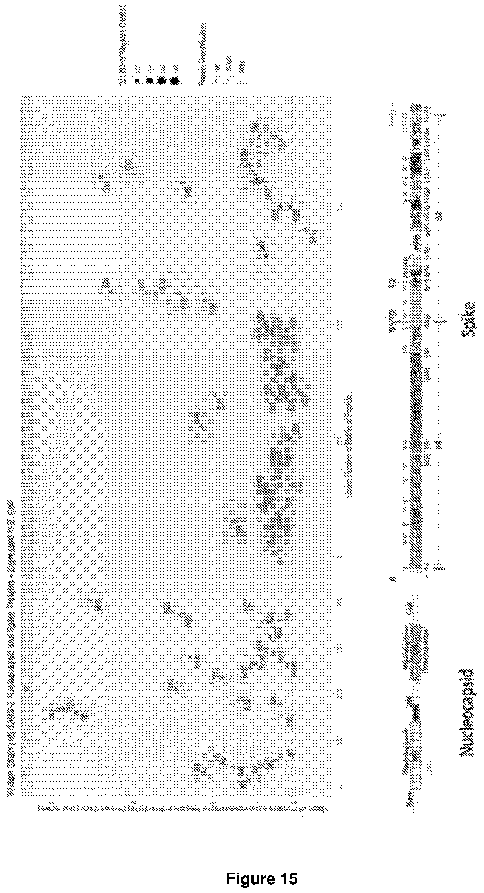

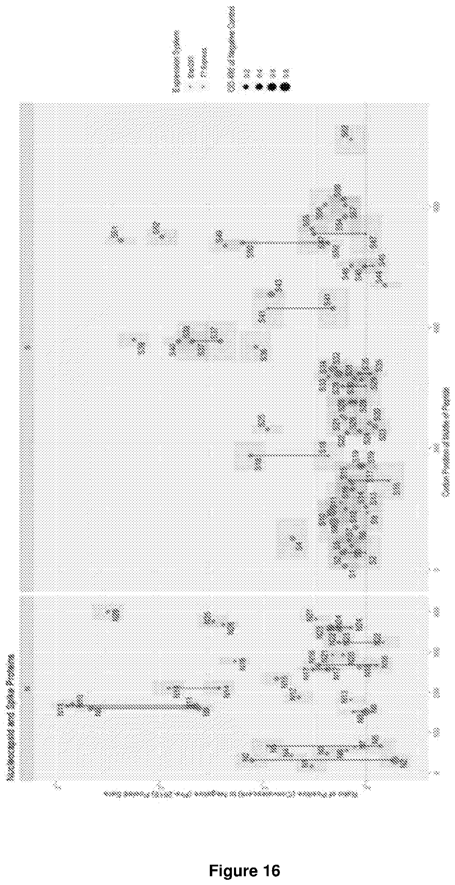

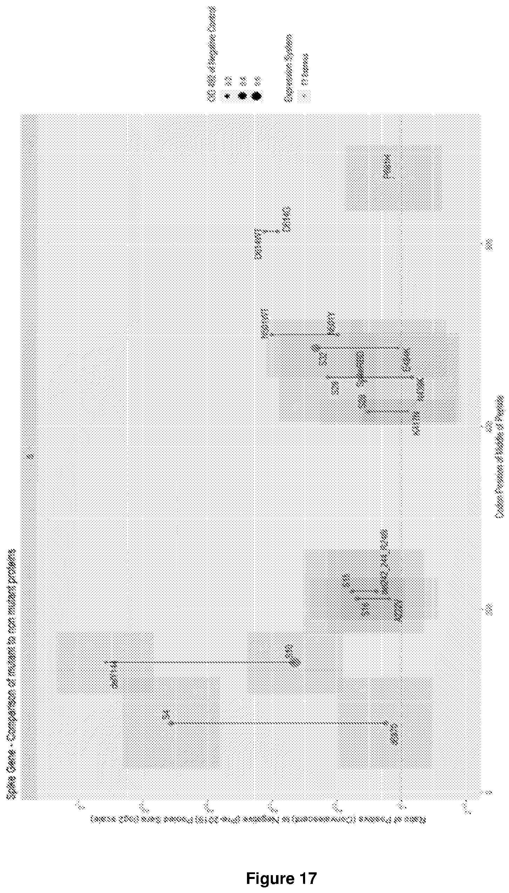

By determining the binding affinity of the pooled samples (see Figure 14), reactive peptides were prioritised using a threshold between the positive and negative signals. See Figure 15. Similarly, differences in binding affinity were observed for peptides purified from bacteria or mammalian cells, indicative of differences in post-translational modification. See Figure 16. Differences in reactivity were observed for mutant peptides. See Figure 17.

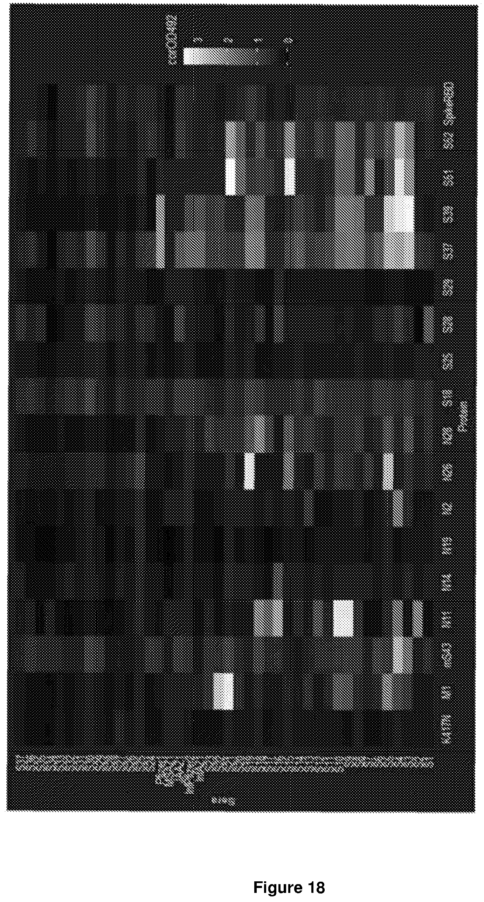

This initial screen was further refined by the characterisation of individual serum samples (see Figure 18), to determine the breadth and strength of antibody response in individual samples.

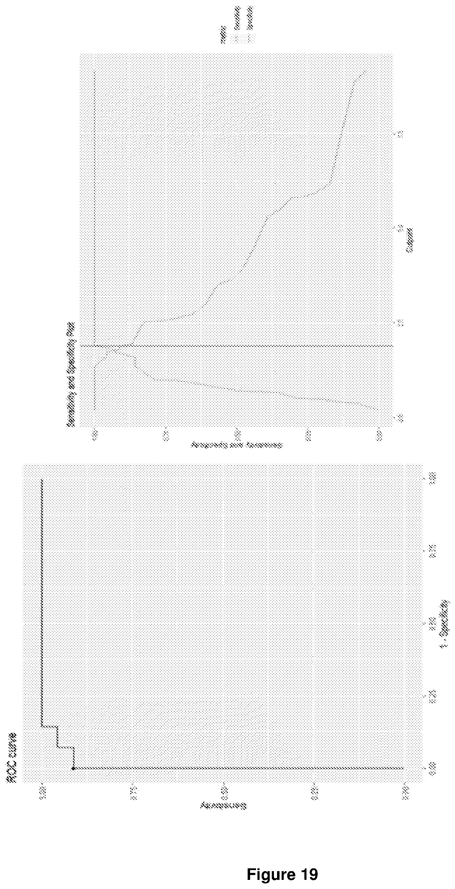

To prioritise peptides the binding affinity was related to a clinical output. By using an algorithm to identify a combination of peptides that provides a good signal to noise ratio with the smallest number of peptides was identified. As a proof of concept this strategy was used to demonstrate that a combination of 5 peptides could discriminate between positive and negative patient samples with 100% sensitivity and 95% specificity. See Figure 19.

Although the disclosure has been described in terms of preferred embodiments as set forth above, it should be understood that these embodiments are illustrative only and that the claims are not limited to those embodiments. Those skilled in the art will be able to make modifications and alternatives in view of the disclosure, which are contemplated as falling within the scope of the appended claims. Each feature disclosed or illustrated in the present specification may be incorporated in any embodiments, whether alone or in any appropriate combination with any other feature disclosed or illustrated herein.

### SEQUENCE IDs

SEQ ID NO: 1
   RITFGGPSDSTGSNQNGERSGARSKQRRPQGLPNN
SEQ ID NO: 2
   QLPQGTTLPKGF
SEQ ID NO: 3
   EGSRGGSQASSRSSSRSRNSSRNSTPGSSR
SEQ ID NO: 4
   NSSRNSTPGSSRGTSPARMAGNGGDAALALLLLDRL
SEQ ID NO: 5
   ALALLLLDRLNQLESKMSGKGQQQQGQTVTKKSA
SEQ ID NO: 6
   AALALLLLDRLNQLE
SEQ ID NO: 7
   KTFPPTEPKKDKKKK
SEQ ID NO: 8
   QALPQRQKKQQTVTLLPAADLDDFSKQLQQSMSSADSTQA
SEQ ID NO: 9
   MADSNGTITVEELKKLLEQ
SEQ ID NO: 10
   HRSYLTPGDSSSGWTAGAAAYYVGYLQPRTFLLKYNENGTITDAVDCALDPLSETKCTLKSFTV EKGIYQTSN
SEQ ID NO: 11
   LTESNKKFLPFQQFGRDIADTTDAVRDPQTLEILDI
SEQ ID NO: 12
   SNKKFLPFQQFGRDIADTTDAVR
SEQ ID NO: 13
   QTQTNSPRRARSVASQ
SEQ ID NO: 14
   LPDPSKPSKRSFIEDLLFNK
SEQ ID NO: 15
   DPLQPELDSFKEELDKYFKNHTSPDVDLGD
SEQ ID NO: 16 
FTTAPAICHDGKAHFPREGVFVSNGTHWFVTQRNFYEPQIITTDNTFVSGNCDVVIGIVNNTVY
DPLQPELD
SEQ ID NO: 17
   PRMCSLMQGSTLPRRSGAAG
SEQ ID NO: 18
   GGGGSPKPSTPPGSSGGGGS
SEQ ID NO: 19
   GGGGS
SEQ ID NO: 20 
SQALPQRQKKQQTVTLLPAADLDDFSKQLQQSMSSADSTQASGSSETPGTAEDGGLQLPQGTTL
PKGFYALASGDGSGASPGLGLTESNKKFLPFQQFGRDIADTTDAVRDPQTLEILDISGSRWPAG
HLPGGLPDPSKPSKRSFIEDLLFNKVTLAG
SEQ ID NO: 21 
QLPQGTTLPKGFGSSGSRWGPAGHLPGGQALPQRQKKQTVTLLPAADLDDFSKQLQQSMSSAD
STQAALASGDAAVASPGLGRITFGGPSDSTGSNQNGERSGARSKQRRPQGLPNNGLGGAAASTG
KTFPPTEPKKDKKKKAGGRSAGGAGNGGDAALALLLLDRLNQLESKMSGLVG
SEQ ID NO: 22 
MADSNGTITVEELKKLLEQGGGGSPKPSTPPGSSGGGGSRITFGGPSDSTGSNQNGERSGARSK
QRRPQGLPNNGGGGSPKPSTPPGSSGGGGSQLPQGTTLPKGFGGGGSPKPSTPPGSSGGGGSAA
LALLLLDRLNQLEGGGGSPKPSTPPGSSGGGGSKTFPPTEPKKDKKKKGGGGSPKPSTPPGSSG
GGGSQALPQRQKKQQTVTLLPAADLDDFSKQLQQSMSSADSTQAGS
SEQ ID NO: 23 
MADSNGTITVEELKKLLEQGGGGSPKPSTPPGSSGGGGSRITFGGPSDSTGSNQNGERSGARSK
QRRPQGLPNNGGGGSPKPSTPPGSSGGGGSQLPQGTTLPKGFGGGGSPKPSTPPGSSGGGGSAA
LALLLLDRLNQLEGGGGSPKPSTPPGSSGGGGSQALPQRQKKQQTVTLLPAADLDDFSKQLQQS
MSSADSTQAGGGGSPKPSTPPGSSGGGGSLTESNKKFLPFQQFGRDIADTTDAVRDPQTLEILD
IGGGGSPKPSTPPGSSGGGGSLPDPSKPSKRSFIEDLLFNKGGGGSPKPSTPPGSSGGGGSFTT
APAICHDGKAHFPREGVFVSNGTHWFVTQRNFYEPQIITTDNTFVSGNCDVVIGIVNNTVYDPL
QPELDSFKEELDKYFKNHTSPDVDLGDGGGGSPKPSTPPGSSGGGGSQTQTNSPRRAPSVASQG
S
SEQ ID NO: 24 
MADSNGTITVEELKKLLEQGGGGSPKPSTPPGSSGGGGSRITFGGPSDSTGSNQNGERSGARSK
QRRPQGLPNNGGGGSPKPSTPPGSSGGGGSQALPQRQKKQQTVTLLPAADLDDFSKQLQQSMSS
ADSTQAGGGGSPKPSTPPGSSGGGGSLTESNKKFLPFQQFGRDIADTTDAVRDPQTLEILDIGG
GGSPKPSTPPGSSGGGGSLPDPSKPSKRSFIEDLLFNKGGGGSPKPSTPPGSSGGGGSDPLQPE
LDSFKEELDKYFKNHTSPDVDLGD
SEQ ID NO: 25 
LTESNKKFLPFQQFGRDIADTTDAVRDPQTLEILDIGGGGSPKPSTPPGSSGGGGSLPDPSKPS
KRSFIEDLLFNKGGGGSPKPSTPPGSSGGGGSQTQTNSPRRAPSVASQGGGGSPKPSTPPGSSG
GGGSFTTAPAICHDGKAHFPREGVFVSNGTHWFVTQRNFYEPQIITTDNTFVSGNCDVVIGIVN
NTVYDPLQPELDSFKEELDKYFKNHTSPDVDLGDGS
SEQ ID NO: 26 
MADSNGTITVEELKKLLEQGGGGSRITFGGPSDSTGSNQNGERSGARSKQRRPQGLPNNGGGGS
QLPQGTTLPKGFGGGGSAALALLLLDRLNQLEGGGGSKTFPPTEPKKDKKKGGGGSQALPQRQ
KKQQTVTLLPAADLDDFSKQLQQSMSSADSTQAGS
SEQ ID NO: 27 
MADSNGTITVEELKKLLEQGGGGSRITFGGPSDSTGSNQNGERSGARSKQRRPQGLPNNGGGGS
QLPQGTTLPKGFGGGGSAALALLLLDRLNQLEGGGGSQALPQRQKKQTVTLLPAADLDDFSKQ
LQQSMSSADSTQAGGGGSLTESNKKFLPFQQFGRDIADTTDAVRDPQTLEILDIGGGGSLPDPS
KPSKRSFIEDLLFNKGGGGSQTQTNSPRRAPSVASQGGGGSFTTAPAICHDGKAHFPREGVFVS
NGTHWFVTQRNFYEPQIITTDNTFVSGNCDVVIGIVNNTVYDPLQPELDSFKEELDKYFKNHTS
PDVDLGDGS
SEQ ID NO: 28 
MADSNGTITVEELKKLLEQGGGGSRITFGGPSDSTGSNQNGERSGARSKQRRPQGLPNNGGGGS
QALPQRQKKQQTVTLLPAADLDDFSKQLQQSMSSADSTQAGGGGSLTESNKKFLPFQQFGRDIA
DTTDAVRDPQTLEILDIGGGGSLPDPSKPSKRSFIEDLLFNKGGGGSDPLQPELDSFKEELDKY
FKNHTSPDVDLGD
SEQ ID NO: 29

LTESNKKFLPFQQFGRDIADTTDAVRDPQTLEILDIGGGGSLPDPSKPSKRSFIEDLLFNKGGG

GSQTQTNSPRRAPSVASQGGGGSFTTAPAICHDGKAHFPREGVFVSNGTHWFVTQRNFYEPQII

TTDNTFVSGNCDVVIGIVNNTVDPLQPELDSFKEELDKYFKNHTSPDVDLGDGS

**Additional sequences**The following table provides a further series of peptides which have been identified using the methods of this disclosure. These peptides are derived from either EBV or SARS-CoV-2 omicron and they have considerable use in diagnostic and prognostic methods, antibody detection and profiling, e.g. 'fingerprinting', vaccine development and variant testing.

| Gene | Gene_Type | Virus | Protein | Start | End | Translation | Length |
|---|---|---|---|---|---|---|---|
| BALF4 | Late Lytic | EBV | gB | 396 | 456 | LTELTTPTSSPP SSPSPPAPSAAR GSTPAAVLRRRR RDAGNATTPVPP TAPGKSLGTLNN P (SEQ ID NO: 30) | 61 |
| BFRF3 | Late Lytic | EBV | capsid protein p18 | 25 | 46 | PKFQELNQNNLP NDVFREAQRS (SEQ ID NO: 31) | 22 |
| EBNA3B | Latent | EBV | EBNA | 473 | 524 | RLEPWQPLPGPQ VTAVLLHEESMQ GVQVHGSMLDLL EKDDEVMEQRVM ATLLP (SEQ ID NO: 32) | 53 |
| EBNA2 | Latent | EBV | EBNA | 370 | 405 | PWRPEPNTSSPS MPELSPVLGLHQ GQGAGDSPTPGP (SEQ ID NO: 33) | 36 |
| EBNA3A | Latent | EBV | EBNA | 881 | 899 | PEWPVQGESGQN VTDHEPR (SEQ ID NO: 34) | 19 |
| EBNA3B | Latent | EBV | EBNA | 16 | 53 | GASGSEDPPDYG DQGNVQQVGSGP ISPEIGPFELSA AS (SEQ ID NO: 35) | 38 |
| EBNALP | Latent | EBV | EBNA | 5 | 58 | SEGPGPTRPGPP GIGPEGPLGQLL RRHRSPSPTRGG QEPRRVRRRVLV QQEEEV   (SEQ ID NO: 36) | 54 |
| BILF1 | Late Lytic | EBV | gp60 | 206 | 224 | VIIIWKLLRTKF GRKPRLI (SEQ ID NO: 37) | 19 |
| BLRF2 | Late Lytic | EBV | capsid protein p23 | 4 | 22 | PRKVRLPSVKAV DMSMEDM (SEQ ID NO: 38) | 19 |
| BBLF1 | Late Lytic | EBV | tegument protein | 43 | 70 | EEGRACGETNEG LEYDEDSENDEL LFLP (SEQ ID NO: 39) | 28 |
| BLRF2 | Late Lytic | EBV | capsid protein p23 | 12 | 161 | GAKGQPSPGEGT RPRESNDPNATR RARSRSRGREAK KVQIS (SEQ ID NO: 40) | 41 |
| BRRF2 | Late Lytic | EBV | tegument protein | 422 | 457 | GSSQAAPSFSSV APVASLSGDLEE EEEGSRESPSLP (SEQ ID NO: 41) | 36 |
| BRRF2 | Late Lytic | EBV | tegument protein | 488 | 526 | RVIGDEDEDGSE DGEFSDLDLSDS DHEGDEGGGAVG GGR (SEQ ID NO: 42) | 39 |
| EBNA1 | Latent | EBV | EBNA | 384 | 446 | PSSQSSSSGSPP RRPPPGRRPFFH PVGEADYFEYHQ EGGPDGEPDVPP GAIEQGPADDPG EGP  (SEQ  ID NO: 43) | 63 |
| BALF4 | Late Lytic | EBV | gB | 254 | 277 | RTNYKIVDYDNR GTNPQGERRAFL (SEQ  ID NO: 44) | 24 |
| LMP1_2 | Latent | EBV | latent membrane protein 1 | 97 | 106 | LWNLHGQALF SEQ ID NO: 45) | 10 |
| M1_BA.5 | Omicron | SARS-CoV-2 | Membrane Protein | 1 | 19 | MANSNGTITVEE LKKLLEE (SEQ ID NO: 46) | 19 |
| M1_BA.1 | Omicron | SARS-CoV-2 | Membrane Protein | 1 | 19 | MADSNGTITVEE LKKLLEE (SEQ ID NO: 47) | 19 |
| M1_BA.2 | Omicron | SARS-CoV-2 | Membrane Protein | 1 | 19 | MAGSNGTITVEE LKKLLEE (SEQ ID NO: 48) | 19 |
| M1_BX | Omicron | SARS-CoV-2 | Membrane Protein | 1 | 19 | MAYSNGTITVEE LKKLLEE (SEQ ID NO: 49) | 19 |

### CLAUSES

1. A peptide or protein comprising or consisting essentially of SEQ ID NO: 9.
2. The peptide or protein of clause 1 for use in a method of diagnosis or for use in medicine.
3. A method of providing a representative peptide, said method comprising:
   obtaining a peptide from a protein;
   comparing a solvent-accessible surface area (SASA) of the peptide with a SASA of the protein; and
   using a result of the comparison to determine whether or not the peptide is a representative peptide.
4. The method of clause 3, wherein a representative peptide is a peptide which retains an epitope and/or the structural, conformational and/or antigenic properties characteristic of a region or domain of the protein.
5. The method of clause 3 or 4, where the SASA of the protein is a SASA of the region or domain of the protein from which the peptide is obtained.
6. The method of any of clauses 3 to 5, wherein two or more peptides are obtained from a protein and wherein the SASA of each of the two or more peptides is compared to a SASA of the protein and the peptide having a SASA closest to the SASA of the peptide is selected as a representative peptide.
7. The method of clause 6, wherein the method comprises determining a protein SASA for each region of the protein from which a peptide has been obtained.
8. A method of identifying stable peptides from a protein, said method comprising:
   obtaining a peptide from a protein;
   comparing a solvent-accessible surface area (SASA) of the peptide with a SASA of a corresponding peptide region within the protein; and
   using a result of the comparison to determine whether or not the peptide is structurally stable relative to the corresponding region within the protein.
9. The method of clause 8, wherein the result of the comparison is a difference between a size of the SASA of the peptide and the SASA of the corresponding peptide region within the protein.
10. The method of clause 8 or 9, wherein the step of obtaining the peptide from the protein comprises fragmenting the protein into a plurality of peptides.
11. The method of clause 10 wherein, for each peptide of the plurality of peptides, a SASA of the peptide is compared with a respective SASA of a corresponding peptide region within the protein.
12. The method of clause 11, comprising comparing the results of each comparison to determine whether one or more peptides of the plurality of peptides is likely to be structurally stable relative to the corresponding region in the protein, wherein comparing the results comprises selecting minima from a plot of each result against a length of a respective, corresponding peptide.
13. The method of any preceding clause, wherein the protein is a SARS-CoV-2 S-protein or a SARS-CoV-2 N protein.
14. Use of the method of any preceding clause to identify thermodynamically stable immunogenic epitopes of SARS-CoV-2.
15. Use of the method of any of clauses 3 to 13 to identify representative or structurally stable peptides from a SARS-CoV-2 protein.
16. A stable or representative peptide identified or obtainable by a method according to any of clauses 3 to 13.
17. A method of protein synthesis comprising:
   - identifying one or more stable peptides using a method corresponding to any of clauses 3 to 13;
   - synthesising DNA sequences corresponding to the one or more peptides; and
   - cloning the DNA sequences into expression vectors.
18. A method of peptide prioritisation comprising using a enzyme-linked immunosorbent assay (ELISA) to identify putatively informative peptides from a plurality of peptides corresponding to structurally stable peptides identified from a protein according to the method of clauses 3 to 13.
19. A computer program comprising instructions which, when the program is executed by a computer, cause the computer to carry out the steps of the method of clause 3.
20. A peptide having a sequence represented by any of SEQ ID NOS: 1-17 or 46-49
21. A peptide having a sequence represented by any of SEQ ID NOS: 30-45
22. A peptide comprising two or more of the peptides provided by clause 18.
23. A peptide according to clause 21, 22 or 23 for use in medicine or in a vaccine.
24. A vaccine or immunogenic composition comprising a peptide obtainable by a method according to any one of clauses 3-13 or as defined in clauses 21 to 23.
25. Use of a peptide according to any one of clauses 21-23 in a method of detecting antibodies in a sample.
26. A method of detecting an antibody in a sample, which antibody binds to a peptide according to any one of clauses 21-23, said method comprising contacting a sample with a peptide according to any one of clauses 21-23 under conditions which permit the formation of peptide/antibody complexes; and detecting antibody/peptide complexes, wherein the detection of an antibody/peptide complex indicates that the sample contains antibodies which bind to a peptide according to any one of clauses 21-23.
27. Use of a peptide according to any one of clauses 21-23 in a diagnostic test or method, or in a method of detecting anti-SARS-CoV-2 antibodies in a sample.
28. Use of a peptide comprising any one of SEQ ID NOS: 1-17, 20-29 or 46-49 in a diagnostic test or method, or in a method of detecting anti-SARS-CoV-2 antibodies in a sample.
29. Use of a peptide comprising any one of SEQ ID NOS: 30-45 in a diagnostic test or method, or in a method of detecting anti-EBV antibodies in a sample.
30. A method of detecting an antibody response, which response is the result of a (natural) infection or vaccination, said method comprising probing a sample for the presence of antibodies which bind to or which have specificity/affinity for a protein or peptide comprising any one or more of SEQ ID NOS: 20, 23, 24, 25, 27, 28 and/or 29.
31. A method of detecting an antibody response, which response is the result of a (natural) infection or vaccination, said method comprising probing a sample for the presence of antibodies which bind to or which have specificity/affinity for a protein or peptide comprising any one or more of SEQ ID NOS: 30-45.
32. A method of detecting an antibody response, which response is the result of a (natural) infection but not vaccination, said method comprising probing a sample for the presence of antibodies which bind to or which have specificity/affinity for a protein or peptide comprising any one or more of SEQ ID NOS: 21, 22 and/or 26.
33. The method of clause 30 or 32, wherein the response is an anti-SARS-CoV-2 response.
34. A method of detecting an anti-SARS-CoV-2 spike antibody response, which response is the result of a (natural) infection or a vaccination, said method comprising probing a sample for the presence of antibodies which bind to or which have specificity/affinity for a protein or peptide comprising any one or more of SEQ ID NOS: 25 or 29.
35. A method of identifying a sample which may contain influenzaA antibodies with cross-reactivity to a SARS-CoV-2 antigen, said method comprising probing a sample for the presence of antibodies which bind to or which have affinity/specificity for a peptide or protein comprising SEQ ID NO: 17.
36. A method of detecting an anti-EBV gB antibody in a sample, which antibody binds to a peptide comprising SEQ ID NOS: 30 and/or 44, said method comprising contacting a sample with a peptide comprising SEQ ID NOS: 30 and/or 44 under conditions which permit the formation of peptide/antibody complexes; and detecting antibody/peptide complexes, wherein the detection of an antibody/peptide complex indicates that the sample contains anti-EBV gB antibodies.
37. A method of detecting an anti-EBV capsid protein p18 antibody in a sample, which antibody binds to a peptide comprising SEQ ID NO: 31, said method comprising contacting a sample with a peptide comprising SEQ ID NO: 31 under conditions which permit the formation of peptide/antibody complexes; and detecting antibody/peptide complexes, wherein the detection of an antibody/peptide complex indicates that the sample contains anti-EBV capsid protein p18 antibodies.
38. A method of detecting an anti-EBV EBNA antibody in a sample, which antibody binds to a peptide comprising any of SEQ ID NOS: 32, 33, 34, 35, 36 and/or 43, said method comprising contacting a sample with a peptide or peptides comprising any of SEQ ID NOS: 32, 33, 34, 35, 36 and/or 43 under conditions which permit the formation of peptide/antibody complexes; and detecting antibody/peptide complexes, wherein the detection of an antibody/peptide complex indicates that the sample contains anti-EBV EBNA antibodies.
39. A method of detecting an anti-EBV gp60 antibody in a sample, which antibody binds to a peptide comprising SEQ ID NO: 37, said method comprising contacting a sample with a peptide comprising SEQ ID NO: 37 under conditions which permit the formation of peptide/antibody complexes; and detecting antibody/peptide complexes, wherein the detection of an antibody/peptide complex indicates that the sample contains anti-EBV gp60 antibodies.
40. A method of detecting an anti-EBV capsid protein p23 antibody in a sample, which antibody binds to a peptide comprising SEQ ID NO: 38 and/or 40, said method comprising contacting a sample with a peptide or peptides comprising SEQ ID NOS: 38 and/or 40 under conditions which permit the formation of peptide/antibody complexes; and detecting antibody/peptide complexes, wherein the detection of an antibody/peptide complex indicates that the sample contains anti-EBV capsid protein p23 antibodies.
41. A method of detecting an anti-EBV tegument protein antibody in a sample, which antibody binds to a peptide comprising SEQ ID NOS: 39, 41 and/or 42, said method comprising contacting a sample with a peptide comprising any of SEQ ID NOS: 39, 41 and/or 42 under conditions which permit the formation of peptide/antibody complexes; and detecting antibody/peptide complexes, wherein the detection of an antibody/peptide complex indicates that the sample contains anti-EBV tegument protein antibodies.
42. A method of detecting an anti-EBV latent membrane protein antibody in a sample, which antibody binds to a peptide comprising SEQ ID NO: 45, said method comprising contacting a sample with a peptide comprising SEQ ID NO: 45 under conditions which permit the formation of peptide/antibody complexes; and detecting antibody/peptide complexes, wherein the detection of an antibody/peptide complex indicates that the sample contains anti-EBV latent membrane protein antibodies.

## Claims

1. A method of providing a representative peptide, said method comprising:
obtaining a peptide from a protein;
comparing a solvent-accessible surface area (SASA) of the peptide with a SASA of the protein; and
using a result of the comparison to determine whether or not the peptide is a representative peptide.

2. The method of claim 1, wherein a representative peptide is a peptide which retains an epitope and/or the structural, conformational and/or antigenic properties characteristic of a region or domain of the protein, and optionally wherein the SASA of the protein is a SASA of the region or domain of the protein from which the peptide is obtained.

3. The method of claim 1 or 2, wherein two or more peptides are obtained from a protein and wherein the SASA of each of the two or more peptides is compared to a SASA of the protein and the peptide having a SASA closest to the SASA of the peptide is selected as a representative peptide, and optionally wherein the method comprises determining a protein SASA for each region of the protein from which a peptide has been obtained.

4. A method of identifying stable peptides from a protein, said method comprising:
obtaining a peptide from a protein;
comparing a solvent-accessible surface area (SASA) of the peptide with a SASA of a corresponding peptide region within the protein; and
using a result of the comparison to determine whether or not the peptide is structurally stable relative to the corresponding region within the protein.

5. The method of claim 4, wherein at least one of:
the result of the comparison is a difference between a size of the SASA of the peptide and the SASA of the corresponding peptide region within the protein;
the step of obtaining the peptide from the protein comprises fragmenting the protein into a plurality of peptides, and/or
for each peptide of the plurality of peptides, a SASA of the peptide is compared with a respective SASA of a corresponding peptide region within the protein, and optionally wherein the method further comprises comparing the results of each comparison to determine whether one or more peptides of the plurality of peptides is likely to be structurally stable relative to the corresponding region in the protein, wherein comparing the results comprises selecting minima from a plot of each result against a length of a respective, corresponding peptide.

6. The method of any preceding claim, wherein the protein is a SARS-CoV-2 S-protein or a SARS-CoV-2 N protein.

7. Use of the method of any preceding claim to identify thermodynamically stable immunogenic epitopes of SARS-CoV-2 and/or representative or structurally stable peptides from a SARS-CoV-2 protein.

8. A stable or representative peptide identified or obtainable by a method according to any of claims 1 to 6.

9. A method of protein synthesis comprising:
- identifying one or more stable peptides using a method corresponding to any of claims 1 to 6;
- synthesising DNA sequences corresponding to the one or more peptides; and
- cloning the DNA sequences into expression vectors.

10. A method of peptide prioritisation comprising using an enzyme-linked immunosorbent assay (ELISA) to identify putatively informative peptides from a plurality of peptides corresponding to structurally stable peptides identified from a protein according to the method of claims 1 to 6.

11. A computer program comprising instructions which, when the program is executed by a computer, cause the computer to carry out the steps of the method of claim 1.

12. A peptide comprising two or more of the peptides provided by claim 10, optionally where said peptide is for use in medicine or in a vaccine .

13. A vaccine or immunogenic composition comprising a peptide obtainable by a method according to any one of claims 1-6 or as defined in claim 12.

14. Use of a peptide according to claim 12 in a method of detecting antibodies in a sample.

15. A method of detecting an antibody in a sample, which antibody binds to a peptide according to claim 12, said method comprising contacting a sample with a peptide according to claim 12 under conditions which permit the formation of peptide/antibody complexes; and detecting antibody/peptide complexes, wherein the detection of an antibody/peptide complex indicates that the sample contains antibodies which bind to a peptide according to claim 12.

16. Use of a peptide according to claim 12 and/or a peptide comprising any one of SEQ **ID** NOS: 1-17, 20-29 or 46-49 in a diagnostic test or method, or in a method of detecting anti-SARS-CoV-2 antibodies in a sample.
